Europäisches Patentamt

**European Patent Office**

Office européen des brevets

(11) Publication number: **0 046 909**

**B1**

(12) **EUROPEAN PATENT SPECIFICATION**

(45) Date of publication of patent specification: **11.06.86**

(21) Application number: **81106324.7**

(22) Date of filing: **13.08.81**

(51) Int. Cl.⁴: **A 61 K 39/42, A 61 K 35/20**

(54) **Pharmaceutical composition.**

(30) Priority: **29.08.80 JP 119312/80**

(43) Date of publication of application:
**10.03.82 Bulletin 82/10**

(45) Publication of the grant of the patent:
**11.06.86 Bulletin 86/24**

(84) Designated Contracting States:
**AT BE CH DE FR GB IT LI SE**

(56) References cited:
**GB-A-1 211 876**
**US-A-3 911 108**

(73) Proprietor: **SENDAI INSTITUTE OF MICROBIOLOGY**
**5-12, Hayama-cho Sendai-shi**
**Miyagi (JP)**

(73) Proprietor: **MITSUBISHI CHEMICAL INDUSTRIES LIMITED**
**5-2, Marunouchi 2-chome Chiyoda-ku Tokyo 100 (JP)**

(72) Inventor: **Ebina, Takusaburo**
**2-12, Hirose-cho**
**Sendai-shi Miyagi (JP)**

(74) Representative: **Patentanwälte TER MEER - MÜLLER - STEINMEISTER**
**Mauerkircherstrasse 45**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to a pharmaceutical composition, which can be used for the treatment of diseases such as intractable diseases including acute gastroenteritis and viral hepatitis, which are caused by virus capable of growing in the digestive tract.

Description of the Prior Art

Heretofore, no effective therapeutic method has been found for intractable neuropathic diseases such as multiple sclerosis, Bechet disease, myasthenia gravis, amyotrophic lateral sclerosis, systemic lupus erythematosus, Parkinson disease, and slow virus infection disease. Corticosteroids such as Prednisolone are the only examples which have been practically used. However, they are accompanied by strong side effects.

US—A—3 911 108 discloses an antibody containing milk produced from a milk delivering animal such as a cow, which milk is intended for use in the treatment of transmissible gastroenteritis a serious disease affecting swine, particularly young piglets. The milk product disclosed in this reference is obtained by immunising a milk producing animal with one or more antigens of viral, bacterial fungal, protozoan or the like origin. This document does not show or suggest the treatment of human beings with the antibody containing milk nor that it is effective against specific human diseases.

GB—A—1 211 876 describes a prophylactic or therapeutic preparation comprising hyper immune bovine colostrum globulin prepared by injecting a pregnant cow with a microorganism. This reference as well does neither show nor suggest the treatment of human beings with the prophylactic or therapeutic preparation disclosed and envisages only the treatment of new-born and young calves.

Summary of the Invention

As a result of a long term virological and immunological study of the above-described diseases, it has now been found that these intractable diseases are autoimmune diseases triggered by virus infections and, to treat such diseases, it is only necessary to avoid the growth of respective virus in a digestive tract. It has also been found that, when a milk animal such as cow or goat, preferably in her pregnancy, is immunized by viruses, lots of antivirus antibody are contained in the milk, particularly colostrum and, upon oral administration of the milk to a patient affected by such intractable disease or diseases, the symptoms can be considerably improved without developing side effects.

Subject matter of the present invention is therefore is a pharmaceutical composition for use in treating a person affected by infantile acute gastroenteritis, comprising as therapeutic agent milk collected from a milk animal which has been immunized by rotavirus, said milk having been subjected to centrifugation to remove bacteria.

An other object of the invention comprises a pharmaceutical composition for use in treating a person affected by viral hepatitis comprising as therapeutic agent milk collected from a milk animal which has been immunized by hepatitis virus, said milk having being subjected to centrifugation to remove bacteria.

Description of the Preferred Embodiments

The therapeutic agent according to this invention and adapted for the treatment of such intractable diseases may be produced, for example, as follows:

An immunization of a milk animal in her 6th—9th month pregnancy is carried out by subcutaneously or intramuscularly inoculating the same animal with the virus which is either rotavirus or an hepatitis virus every 7 to 14 days and 3 to 6 times in total. The amount of virus to be inoculated is not specifically limited but its upper limit is determined depending upon side effects which may be caused by the inoculation. Colostrum is collected, preferably, for 1 to 3 days after the birth and subjected to centrifugation to remove bacteria and to provide the therapeutic agent for the above-described diseases.

The therapeutic agent for infantile acute gastroenteritis can be produced by an inoculation of rotavirus. On the other hand, the therapeutic agent for viral hepatitis can be prepared by an inoculation of A-type, B-type or Non-A and Non-B type hepatitis virus. It is also possible to use vaccine in place of the virus per se.

The pharmaceutical composition according to this invention providing the effect of alleviating the state of a disease caused by virus may be applied in any suitable manner, but an oral administration is preferred as it permits intestinal absorption. Thus, the milk is orally administered as it is or after converting it to powder milk through dehydration.

The dosage administered will be dependent upon the age, condition and weight of the patient, the state of disease, kind of concurrent treatment if any, frequency of treatment, and the nature of the effect desired.

Generally, a daily dosage measured as milk will be from about 0.1—10 ml per kg of body weight. Normally, from 1—2 ml per kg per day, in one or more applications per day is effective to obtain the desired result.

The pharmaceutical composition of this invention can be employed in dosage from such as tablets, capsules, powder packets, granules, or liquid solutions, suspension, or elixirs, for oral administration.

Besides the therapeutic agent, the composition will contain a solid or liquid non-toxic pharmaceutical carrier for the active ingredient. In one embodiment of a composition, the solid carrier can be a capsule of the ordinary gelatin type.

In another embodiment, the active ingredient can be tableted with or without adjuvants, or put into powder packets.

## Claims

1. Pharmaceutical composition for use in treating a person affected by infantile acute gastroenteritis, characterized by comprising as therapeutic agent milk collected from a milk animal which has been immunized by rotavirus, said milk having been subjected to centrifugation to remove bacteria.

2. Pharmaceutical composition for use in treating a person affected by viral hepatitis, characterized by comprising as therapeutic agent milk collected from a milk animal which has been immunized by hepatitis virus, said milk having been subjected to centrifugation to remove bacteria.

3. The pharmaceutical composition according to claims 1 and 2, characterized by comprising as therapeutic agent milk collected from a milk animal which has been immunized by the virus in a form of a virus vaccine.

4. The pharmaceutical composition according to one of the preceding claims, characterized by comprising as therapeutic agent milk collected from a milk animal which has been immunized with the virus or a vaccine of the same virus while in her pregnancy.

5. The pharmaceutical composition according to claim 4, characterized in that the milk is colostrum.

6. The pharmaceutical composition according to one of the preceding claims, characterized by comprising as therapeutic agent milk collected from a cow being used as milk animal.

7. The pharmaceutical composition according to one of the preceding claims, characterized in that it further contains a solid or liquid nontoxic pharmaceutical carrier, diluent and/or adjuvant.

## Revendications

1. Composition pharmaceutique pour l'emploi dans le traitement d'un sujet atteint de gastro-entérite aiguë infantile caractérisée en ce qu'elle comprend comme agent thérapeutique le lait provenant d'un animal laitier que l'on a immunisé par un rotavirus, ledit lait ayant été soumis à une centrifugation pour éliminer les bactéries.

2. Composition pharmaceutique pour l'emploi dans le traitement d'un sujet atteint d'hépatite virale caractérisée en ce qu'elle comprend comme agent thérapeutique le lait provenant d'un animal laitier qui a été immunisé par le virus de l'hépatite, ledit lait ayant été soumis à une centrifugation pour éliminer les bactéries.

3. La composition pharmaceutique selon les revendications 1 et 2 caractérisée en ce qu'elle comprend comme agent thérapeutique le lait provenant d'un animal laitier qui a été immunisé par le virus sous forme d'un vaccin viral.

4. La composition pharmaceutique selon l'une quelconque des revendications précédentes caractérisée en ce qu'elle comprend comme agent thérapeutique le lait provenant d'un animal laitier qui a été immunisé par le virus ou un vaccin de même virus alors qu'il était gravide.

5. La composition pharmaceutique selon la revendication 4 caractérisée en ce que le lait est du colostrum.

6. La composition pharmaceutique selon l'une quelconque des revendications précédentes caractérisée en ce qu'elle comprend comme agent thérapeutique le lait provenant d'une vache utilisée comme animal laitier.

7. La composition pharmaceutique selon l'une quelconque des revendications précédentes caractérisée en ce qu'elle contient de plus un support, diluant et/ou adjuvant pharmaceutique non toxiques solides ou liquides.

## Patentansprüche

1. Pharmazeutische Zubereitung zur Behandlung von Personen, die von infantiler akuter Gastroenteritis befallen sind, dadurch gekennzeichnet, daß sie als therapeutischen Wirkstoff die Milch eines Milchtiers enthält, welches mit Rotavirus immunisiert worden ist, welche Milch zur Entfernung von Bakterien zentrifugiert worden ist.

2. Pharmazeutische Zubereitung zur Behandlung von Personen, die von Virushepatitis befallen sind, dadurch gekennzeichnet, daß sie als therapeutischen Wirkstoff die Milch eines Milchtiers enthält, welches mit Hepatitisvirus immunisiert worden ist, welche Milch zur Entfernung von Bakterien zentrifugiert worden ist.

3. Pharmazeutische Zubereitung nach den Ansprüchen 1 und 2, dadurch gekennzeichnet, daß sie als therapeutischen Wirkstoff die Milch eines Milchtiers enthält, welches mit dem Virus in Form eines Virus-Impfstoffs immunisiert worden ist.

4. Pharmazeutische Zubereitung nach einem der vorhergehenden Ansprüche, dadurch gekennzeichnet, daß sie als therapeutischen Wirkstoff die Milch eines Milchtiers umfaßt, welches während der Trächtigkeit mit dem Virus oder einem Impfstoff des gleichen Virus immunisiert worden ist.

5. Pharmazeutische Zubereitung nach Anspruch 4, dadurch gekennzeichnet, daß sie als Milch Kolostrum enthält.

6. Pharmazeutische Zubereitung nach einem der vorhergehenden Ansprüche, dadurch gekennzeichnet, daß sie als therapeutischen Wirkstoff die Milch einer als Milchtier verwendeten Kuh enthält.

7. Pharmazeutische Zubereitung nach einem der vorhergehenden Ansprüche, dadurch gekennzeichnet, daß sie zusätzlich einen festen oder flüssigen, nichttoxischen pharmazeutischen Träger, ein Verdünnungsmittel und/oder einen Hilfsstoff enthält.